**Europäisches Patentamt**

**European Patent Office**

**Office européen des brevets**

(11) Publication number : **0 502 719 A1**

# (12) EUROPEAN PATENT APPLICATION

(21) Application number : **92301869.1**

(22) Date of filing : **04.03.92**

(51) Int. Cl.⁵ : **A01N 63/00,** A01N 65/00,
C12N 15/29, C12N 15/05,
C12N 5/04, A61K 37/02

(30) Priority : **04.03.91 US 664269**

(43) Date of publication of application :
**09.09.92 Bulletin 92/37**

(84) Designated Contracting States :
**AT BE CH DE DK ES FR GB GR IT LI LU NL SE**

(71) Applicant : **PIONEER HI-BRED
INTERNATIONAL, INC.
700 Capital Square 400 Locust Street
Des Moines Iowa 50309 (US)**

(72) Inventor : **Duvick, Jonathan
1707 38th Street, Des Moines
Polk County, Iowa 50310 (US)**
Inventor : **Rood, Tracy
4333 Allison Avenue, Des Moines
Polk County, Iowa 50310 (US)**

(74) Representative : **Goldin, Douglas Michael et al
J.A. KEMP & CO. 14, South Square Gray's Inn
London WC1R 5LX (GB)**

(54) **Plant polypeptides with inhibitory activity towards pathogenic microorganisms.**

(57) Certain polypeptides derived from plants, Bandeiraea simplicifolia lectin II and chymopapain, have been found to have potent antimicrobial properties. Plant resistance to diseases caused by plant pathogens which are susceptible to these proteins can be produced by inserting into the cells of a plant a gene whose expression causes production a selected protein in antimicrobial amounts.

EP 0 502 719 A1

## Technical Field

This invention relates to materials and methods for killing and inhibiting fungal and other pathogens of plants, humans and animals, and materials and methods for directly imparting disease resistance to plants.

## Background of the Invention

There is a great interest in the development of new microbicides that do not have adverse effects on the host organism but have high potency against pathogens. One area of interest in locating microbicidally active compounds is naturally occurring compounds, because they can be readily detoxified by natural mechanisms in the environment and in humans and other animals.

## Humans and Lower Animals

Bacteria and fungi are the cause of many diseases in humans and lower animals. These diseases can range from minor irritations such as athlete's foot to serious or even fatal systemic infections. Often the medications used in treating systemic infections, particularly those used in treating systemic fungal infections, cause serious side affects in the treated human or animal. Therefore there is a continuing need in the fields of human and veterinary medicine for the development of new and effective antiinfective compounds.

## Plants

Numerous fungi and bacteria are not only pathogens in humans and lower animals, but are also serious pests of agricultural crops. One method of controlling plant diseases in the past has been to apply antimicrobial organic or semiorganic chemicals to crops. This method has numerous, art-recognized problems. A more recent method of control of microorganism pests has been the use of biological control organisms which are typically natural competitors or inhibitors of the troublesome microorganisms. However, it is difficult to apply biological control organisms to large areas, and even more difficult to cause those living organisms to remain in the treated area for an extended period. Still more recently, techniques in recombinant DNA have provided the opportunity to insert into plant cells cloned genes which express proteins in the plant cells. Some proteins are known to have antimicrobial activity. However, this technology has given rise to additional concerns about eventual microbial resistance to well-known, naturally occurring antimicrobials, particularly in the face of heavy selection pressure, which may occur in some areas. Thus, a continuing need exists to identify additional naturally occurring antimicrobial compounds which can be formed by plant cells directly by translation of a single structural gene.

European Patent Application 204,590, based upon U.S. Patent application Serial No. 725,368, describes a method of genetically modifying a plant cell to control expression of heterologous foreign structure genes. In the method, the plant cell is transformed to contain a pRi T-DNA promoter and a heterologous foreign structural gene, the promoter and the structural gene being in such position and orientation with respect to one another that the structural gene is expressible in a plant cell under control of the promoter.

Likewise, European Patent Application 186,425, based upon U.S. patent application Serial No. 685,824, describes a recombinant DNA expression vector which comprises (a) a transcription unit, flanked by T-DNA border sequences, which comprises a promoter and associated amino terminal region encoding sequences and a terminator signal sequence in which the sequences are derived from one or more genes which are naturally expressed in a plant cell, and (b) an antibiotic resistance gene-encoding sequence located between the promoter and associated amino-terminal region-encoding sequence end the terminator sequence and (c) a DNA fragment containing a replicon that is functional in Agrobacterium.

PCT application 8807087, based upon U.S. patent application 168,109, discloses a recombinant virus expression system comprising a Heliothis polyhedrin promoter and a nucleotide sequence encoding a heterologous peptide or protein, which may have antimicrobial activity.

## Summary of the Invention

According to this invention, two polypeptides derived from plants, Bandeiraea lectin II and chymopapain, have been determined to have potent antimicrobial activity against many common pathogens. This invention thus relates broadly to compositions containing these polypeptides, and methods of using these polypeptides and compositions containing them against human, animal and plant pathogens. Since it is presently considered feasible and desirable to genetically engineer plants to express proteins which, inter alia, impart resistances to diseases and other pests, this invention also relates broadly to methods of creating plants with disease resistance by virtue of expressing one or both of these polypeptides, and to cells and plants created thereby.

## Disclosure of the Invention

It has now been determined that a certain proteins which occur naturally in minute amounts in plants, Bandeiraea simplicifolia lectin II "Bandeiraea II" and a thiol protease from papaya latex, chymopapain, have antimicrobial activity against many common pathogens. Bandeiraea II is most active against the pathogenic fungus Fusarium moniliforme, an ear and stalk pathogen of maize and sorghum, the tobacco leaf pathogen, Alternaria longipes, and Aspergillus flavus. Chymopapain is most active against Fusarium graminearum, Alternaria longipes and Fusarium moniliforme.

Thus, this invention provides a method for killing and inhibiting susceptible pathogens, including microorganisms selected from the group consisting of Fusarium graminearum, Alternaria longipes and Fusarium moniliforme, comprising introducing into the environment of the organisms an antimicrobial amount of Bandeiraea II, chymopapain or a combination thereof.

This invention also provides compositions of matter for the treatment and prevention of diseases in humans, lower animals, and plants caused by susceptible organisms, comprising one or both of the foregoing polypeptides in combination with a non-toxic carrier.

## Plants

The polypeptides employed in this invention, Bandeiraea II and chymopapain, can be effectively applied to plants afflicted with susceptible microorganisms by any convenient means, including spray, creams, dust or other formulation common to the antimicrobial arts. The Bandeiraea II or chymopapain can also be incorporated systemically into the tissues of a treated plant so that in the course of infesting the plant the pathogens will be exposed to antimicrobial amounts of Bandeiraea II or chymopapain. One method of doing this is to incorporate the Bandeiraea II or Chymopapain in a non-phytotoxic vehicle which is adapted for systemic administration to the susceptible plants. This method is commonly employed with fungicidal materials such as captan and is well within the purview of one of ordinary skill in the art of plant fungicide formulation. However, since the genes which code for Bandeiraea II and chymopapain can be isolated, cloned, inserted into an appropriate expression cassette, and introduced into cells of a susceptible plant species, an esepcially preferred embodiment of this method involves inserting into the genome of the plant a DNA sequence coding for Bandeiraea II or chymopapain in proper reading frame, together with transcription initiator and promoter sequences active in the plant. Transcription and translation of the DNA sequence under control of the regulatory sequences causes expression of Bandeiraea II or chymopapain protein sequence at levels which provide an antimicrobial amount of Bandeiraea II or chymopapain in the tissues of the plant which are normally infected by the pathogens.

The plant is preferably a plant susceptible to infection and damage by one or more of Fusarium graminearum, Fusarium moniliforme, Aspergillus flavus, Alternaria longipes, Sclerotinia sclerotiorum, and Sclerotinia trifoliorum. These include corn (Zea mays) and sorghum (Sorghum bicolor). However, this is not to be construed as limiting, inasmuch as these two species are among the most difficult commercial crops to reliably transform and regenerate, and these pathogens also infect certain other crops. Thus the methods of this invention are readily applicable via conventional techniques to numerous plant species, if they are found to be susceptible to the plant pathogens listed hereinabove, including, without limitation, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersionn, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum, and Datura.

Preferred plants that are to be transformed according to the methods of this invention are cereal crops, including maize, rye, barley, wheat, sorghum, oats, millet, rice, triticale, sunflower, alfalfa, rapeseed and soybean.

The DNA sequence which when expressed imparts antimicrobial activity is a structural gene which codes for Bandeiraea II or chymopapain as described herein. In general, since one object of the invention is to confer resistance to a microorganism to which the plant is susceptible, the Bandeiraea II or chymopapain will not be native to the plant.

The DNA sequences which code for Bandeiraea II or chymopapain in the practice of this invention can be obtained by conventional techniques and the gene can then be removed from the native genome by use of appropriate restriction enzymes and spliced into a selected plant expression cassette. Alternatively, the purified Bandeiraea II or chymopapain polypeptides can be sequenced in their entirety using known methods, and synthetic DNA sequences can then be prepared which code for the appropriate sequence of amino acids, and this synthetic DNA sequence can be inserted into an appropriate plant expression cassette.

Likewise, numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong constitutive promoter sequence at one end to cause the gene to be transcribed at a high frequency, and a poly-A recognition sequence at the other end for proper processing and transport of the messenger RNA. An example of such a preferred (empty) expression cassette into which the cDNA of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endonuclease recognition sites which may be cut in a predictable fashion by use of the appropriate enzyme. Such vectors are preferably constructed to include additional structural gene sequences imparting antibiotic or herbicide resistance, which then serve as markers to identify and separate transformed cells. Preferred markers/selection agents include kanamycin, chlorosulfuron, phosphonothricin, hygromycin and methotrexate. A cell in which the foreign genetic material in a vector is functionally expressed has been "transformed" by the vector and is referred to as a "transformant".

A particularly preferred vector is a plasmid, by which is meant a circular double-stranded DNA molecule which is not a part of the chromosomes of the cell.

As mentioned above, both genomic and cDNA encoding the gene of interest may be used in this invention. The vector of interest may also be constructed partially from a cDNA clone and partially from a genomic clone. When the gene of interest has been isolated, genetic constructs are made which contain the necessary regulatory sequences to provide for efficient expression of the gene in the host cell. According to this invention, the genetic construct will contain (a) a first genetic sequence coding for the protein or trait of interest and (b) one or more regulatory sequences operably linked on either side of the structural gene of interest. Typically, the regulatory sequences will be selected from the group comprising of promoters and terminators. The regulatory sequences may be from autologous or heterologous sources.

Promoters that may be used in the genetic sequence include nos, ocs and CaMV promoters.

An efficient plant promoter that may be used is an overproducing plant promoter. Overproducing plant promoters that may be used in this invention include the promoter of the small sub-unit (ss) of the ribulose-1,5-biphosphate carboxylase from soybean (Berry-Lowe et al., J. Molecular and App. Gen., 1:483-498 (1982)), and the promoter of the cholorophyll a-b binding protein. These two promoters are known to be light-induced, in eukaryotic plant cells (see, for example, Genetic Engineering of Plants, An Agricultural Perspective, A. Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)).

The expression cassette comprising the structural gene for Bandeiraea II or chymopapain operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as E. coli, S. typhimurium, and Serratia marcescens. Eukaryotic hosts such as yeast or filamentous fungi may also be used in this invention. Since these hosts are also microorganisms, it will be essential to ensure that plant promoters which do not cause expression of the Bandeiraea lectin or chymopapain in bacteria are used in the vector.

The isolated cloning vector will then be introduced into the plant cell using any convenient technique, including electroporation (in protoplasts), retroviruses, bombardment, and microinjection into cells from monocotyledonous or dicotyledonous plants in cell or tissue culture to provide transformed plant cells containing as foreign DNA at least one copy of the DNA sequence of the plant expression cassette. Preferably, the monocotyledonous species will be selected from maize, sorghum, wheat or rice, and the dicotyledonous species will be selected from soybean, alfalfa, tobacco or tomato. Using known techniques, protoplasts can be regenerated and cell or tissue culture can be regenerated to form whole fertile plants which carry and express the gene for Bandeiraea II lectin or chymopapain. Accordingly, a highly preferred embodiment of the present invention is a transformed maize plant, the cells of which contain as foreign DNA at least one copy of the DNA sequence of an expression cassette of this invention.

Finally, this invention provides methods of imparting resistance to diseases caused by microorganisms selected from Fusarium graminearum, Fusarium moniliforme, Diplodia maydis, Colletototrichum graminicola, Verticillium alboatrum, Phytophthora megaspermae f.sp. glycinea, Macrophomina phaseolina, Diaporthe phaseolorum caulivora, Sclerotinia sclerotiorum, Sclerotinia trifoliorum, Aspergillus flavus to plants of a susceptible taxon, comprising the steps of:

a) culturing cells or tissues from at least one plant from the taxon,

b) introducing into the cells or tissue culture at least one copy of an expression cassette comprising a structural gene for Bandeiraea II or chymopapain, operably linked to plant regulatory sequences which cause the expression of the Bandeiraea II or chymopapain structural gene in the cells, and

c) regenerating disease-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the Bandeiraea II or chymopapain structural gene and associated regulatory sequences via crossing and backcrossing. Such intermediate methods will comprise the further steps of

a) sexually crossing the disease-resistant plant with a plant from the disease-susceptible taxon;

b) recovering reproductive material from the progeny of the cross; and

c) growing disease-resistant plants from the reproductive material. Where desirable or necessary, the agronomic characteristics of the susceptible taxon can be substantially preserved by expanding this method to include the further steps of repetitively:

a) backcrossing the disease-resistant progeny with disease-susceptible plants from the susceptible taxon; and

b) selecting for expression of antimicrobial activity (or an associated marker gene) among the progeny of the backcross, until the desired percentage of the characteristics of the susceptible taxon are present in the progeny along with the gene imparting antimicrobial activity.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incorporated into Agrobacterium tumefaciens, which can then be used to transfer the vector into susceptible plant cells, primarily from dicotyledonous species. Thus, this invention provides a method for imparting antimicrobial activity and disease resistance in Agrobacterium tumefaciens-susceptible dicotyledonous plants in which the expression cassette is introduced into the cells by infecting the cells with Agrobacterium tumefaciens, a plasmid of which has been modified to include a plant expression cassette of this invention.

## Human and Veterinary Pharmaceutical Use

This invention also provides methods of treating and preventing infection by susceptible organisms in a human or lower animal host in need of such treatment, which method comprises administration to the human or lower animal host in need of such treatment a therapeutically effective amount of a polypeptide of this invention or a composition containing one or both of the polypeptides. The polypeptides of the present invention may be administered parenterally, by inhalation spray, rectally or topically in dosage unit formulations containing conventional nontoxic pharmaceutically acceptable carriers, adjuvants and vehicles as desired. The term parenteral as used herein includes subcutaneous, intravenous, intramuscular, intraarticular and intrathecal injection and infusion techniques. As with other polypeptides, the polypeptides of this invention are not known to be active orally.

Total daily dose of the compounds of this invention administered to a host in single or divided doses may be in amounts, for example, of from 1 to 2000 mg/kg body weight daily and more usually 50 to 500 mg/kg. Dosage unit compositions may contain such amounts or fractions or submultiples thereof as appropriate to make up the daily dose. It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

This invention also provides pharmaceutical compositions in unit dosage form, comprising an effective amount of a compound of this invention in combination with a conventional pharmaceutical carrier. As used herein, the term "pharmaceutical carrier" means a solid or liquid filler, diluent or encapsulating material. Some examples of the materials which can serve as pharmaceutical carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such peanut oil, cottonseed oil, safflower oil, sesame oil, olive oil, corn oil and soybean oil; polyols such as propylene glycol, glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution, ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, release agents, coating agents, and perfuming agents and preservatives can also be present in the compositions, according to the desires of the formulator. The amount of active ingredient that may be combined with the carrier materials to produce a single dosage form will vary depending upon the host treated and the particular mode of administration.

By "therapeutically effective amount" herein is meant an amount of either polypeptide or combination thereof sufficient to provide antimicrobial activity so as to alleviate or prevent infection by susceptible organisms in the human or lower animal being treated at a reasonable benefit/risk ratio attendant with any medical treatment.

## Industrial Applicability

The following description further exemplifies the compositions of this invention and the methods of making and using them. However, it will be understood that-other methods, known by those of ordinary skill in the art to be equivalent, can also be employed.

## Example

## Microorganism Susceptibility to Bandeiraea II and Chymopapain

In spore germination and hyphal growth assays against plant pathogenic fungi, Bandeiraea lectin II at 3 micromolar has been found in some experiments to have inhibitory activity against Alternaria longipes, Fusarium moniliforme, and Aspergillus flavus. Other fungi, including Fusarium graminearum, Sclerotinia sclerotiorum and Sclerotinia trifoliorum, were slightly inhibited at that concentration in certain experiments. Chymopapain has been found to have strong inhibitory activity at 3 micromolar against Fusarium graminearum, and moderate activity against Alternaria longipes, Aspergillus flavus, and Fusarium moniliforme.

Approximately 200-250 conidia or approximately 20-30 mycelial fragments were incubated in 90 microliters of culture medium in microtiter plate wells. The culture medium was a growth medium of PH 5.5 containing 0.25% yeast extract, 0.1% casein hydrolyzate, 1% glucose, 0.25% calcium nitrate, 0.05% potassium phosphate, and 0.0625% magnesium sulfate, 0.038% sodium chloride, and 2.5 µM sodium phosphate. The Bandeiraea II lectin or the chymopapain at the test concentration was added in 10 microliters in water or buffer, and the plates were incubated from one to three days at 28 degrees C. At the end of the incubation period, the wells were examined and mycelial growth was scored versus control wells as follows:

0 - No inhibition
1 - Slight Inhibition
2 - Moderate Inhibition
3 - Almost Complete Inhibition
4 - Complete Inhibition

Results of one such study are indicated in Tables 1 and 2.

Table 1

## Level I Results of Antifungal Screening

Data represent antifungal scores obtained in two or three experiments, one to two reps per experiment.

| | ALO | AFL | FGR | FMO | SSC | STR |
|---|---|---|---|---|---|---|
| *Bandeiraea simplicifolia* lectin II (3 uM = 338 ug/ml) | 3,2 | 1.5,1.5 | 1,1 | 4,4 | 0,0 | 0,0 |
| •from Vector (L-1210) | 2.5 | 2.5 | 2 | 1 | 1.5,3 | 0,3 |
| •isolated from seeds of tropical tree | 1,1 | 1,0 | 0,0 | 0,0 | | |

•tetrameric glycoprotein with 4 subunits
•recognizes exclusively alpha- or beta-linked
N-acetylglucosamine residues on the nonreducing
end of oligosaccharides
•homogeneous, salt-free
•ref: Histochemical Journal, 18: 589-596 (1986)

| | ALO | AFL | FGR | FMO | SSC | STR |
|---|---|---|---|---|---|---|
| Chymopapain (3 uM = 105 ug/ml) | 2.5,2.5 | 1,1 | 4,4 | 2.25,2.5 | 0,0 | 0,0 |
| •from Sigma (C8526) | 2,2 | 2,2 | 4,4 | 2.5,2.5 | 0,0 | 0,0 |

•isolated from papaya latex, chromatographically purified
•thiol protease
•~75% protein; balance L-cysteine and EDTA
•substantially free of papain, lysozyme and peptidase A
•Chemical Abstract Survey #9001-09-6

Key:     ALO = *Alternaria longipes*
AFL = *Aspergillus flavus*
FGR = *Fusarium graminearum*
FMO = *Fusarium moniliforme*
SSC = *Sclerotinia sclerotiorum*
STR = *Sclerotinia trifoliorum*

EP 0 502 719 A1

## Table 2

## Level II results

Data represent minimal effective concentration (MEC), which is the lowest concentration to attain a score of "2" or greater on a 0-4 scale.

|  | ALO | AFL | FGR | FMO | SSC | STR |
|---|---|---|---|---|---|---|
| *Bandeiraea simplicifolia* lectin II |  |  |  | 2.7 uM |  |  |
| Chymopapain | 3 uM | 3 uM | 1 uM | 3 uM | 9 uM | 9 uM |

Key:
ALO = *Alternaria longipes*
AFL = *Aspergillus flavus*
FGR = *Fusarium graminearum*
FMO = *Fusarium moniliforme*
SSC = *Sclerotinia sclerotiorum*
STR = *Sclerotinia trifoliorum*

EP 0 502 719 A1

In other studies, Fusarium graminearum was found to be the most sensitive of the fungi to the action of chymopapain, showing noticeable inhibition at 1.0 micromolar. Sclerotinia trifoliorum and Sclerotinia sclerotiorum were the most resistant, showing inhibition at 9 micromolar, Bandeiraea II was found to be active only against Fusarium moniliforme.

In summary, Bandeiraea II or chymopapain is inhibitory to a wide range of pathogens, although inhibition profiles vary widely from pathogen to pathogen.

All percentages herein are by weight, except as otherwise indicated. The term "microorganisms" as used herein includes bacteria and fungi. The terms "bacteria" and "bacterial infection" are used herein in their usual context. By "fungi" herein are meant the higher protists, including the phycomycetes, the ascomycetes and basidiomycetes, as well as other protista commonly referred to as "yeasts" or "molds".

## Claims

1. A method for killing or inhibiting the growth of a microorganism which is susceptible to a polypeptide which is: Bandeiraea II lectin, chymopapain, or mixtures thereof; comprising introducing into the environment of the microorganisms an antimicrobial amount of the polypeptide or mixture.

2. A method according to claim 1 wherein the environment is the locus of a plant.

3. A method according to claim 2 wherein the environment is the tissues of a living plant.

4. A method according to claims 2 or 3 wherein the plant is a monocotyledonous species selected from wheat, rice, and sorghum.

5. A method according to any one of the preceding claims wherein the microorganism is the pathogen Fusarium graminearum, Fusarium moniliforme, Aspergillus flavus, Alternaria longipes, Sclerotinia sclerotiorum, or Sclerotinia trifoliorum.

6. A method according to claim 5 for protecting a plant against infection comprising inserting into the genome of the plant a foreign DNA sequence coding for Bandeiraea II lectin, chymopapain, or both, in proper reading frame relative to transcription initiator and promoter sequences active in the plant to cause expression of the polypeptide or polypeptides at antimicrobial levels in the tissues of the plant which are normally infected by the pathogens.

7. A method according to claim 6 wherein the foreign DNA sequence is inserted by the steps:
   a) culturing cells or tissues from the plant
   b) introducing into the cells or tissue culture at least one copy of an expression cassette comprising a sequence coding for a Bandeiraea II lectin, chymopapain, or both, and
   c) regenerating protected whole plants from the cell or tissue culture.

8. A method according to claim 7 which comprises the further step of sexually or clonally reproducing the whole plant in such manner that at least one copy of the sequence provided by the expression cassette is present in the cells of progeny of the reproduction.

9. A method according to claim 7 in which the expression cassette is introduced into the cells by electroporation.

10. A method according to claim 7 in which the expression cassette is introduced into the cells by microparticle bombardment.

11. A method according to claim 7 in which the expression cassette is introduced into the cells by microinjection.

12. A method according to claim 7 for providing resistance to microorganisms in Agrobacterium tumefaciens susceptible dicotyledonous plants in which the expression cassette is introduced into the cells by infecting the cells with Agrobacterium tumefaciens, a plasmid of which has been modified to include the expression cassette.

13. A method of imparting resistance to diseases caused by pathogens selected from Fusarium graminearum, Fusarium, moniliforme, Aspergillus flavus, Alternaria, longipes, sclerotinia sclerotiorum, and Sclerotinia trifoliorum, to plants of a taxon susceptible to those diseases, comprising the steps of:

a) selecting a fertile, disease resistant plant prepared by the method of any one of claims 6 to 12 from a sexually compatible plant;

b) sexually crossing the disease resistant plant with a plant from the disease susceptible taxon;

c) recovering reproductive material from the progeny of the cross; and

d) growing protected plants from the reproductive material.

14. A method according to any one of claims 6 to 12 for imparting disease resistance and antimicrobial activity in a taxon consisting of substantially homozygous plants, which comprises the further steps of repetitively:

a) backcrossing the disease resistant progeny with substantially homozygous, disease susceptible plants from the taxon; and

b) selecting for expression of disease resistance and antimicrobial activity along with the other desired characteristics of the susceptible taxon from among the progeny of the backcross, until the desired percentage of the characteristics of the susceptible taxon are present in the progeny along with disease resistance and antimicrobial activity.

15. A DNA clone from the genome of a plant which codes substantially solely for Bandeiraea II lectin or chymopapain.

16. An expression cassette comprising a DNA clone according to claim 15, operably linked to plant regulatory sequences which cause the expression of the DNA clone in plant cells which do not naturally contain the clone.

17. An expression cassette comprising a DNA clone according to claim 15 operably linked to bacterial sequences which cause the replication of the DNA clone in bacterial cells.

18. Bacterial cells containing as a foreign plasmid at least one copy of an expression cassette according to claim 17.

19. Transformed plant cells containing as foreign DNA at least one copy of the DNA sequence of an expression cassette according to claim 16.

20. Transformed cells according to claim 19, further characterized in being cells of a monocotyledonous species, preferably maize, sorghum, wheat or rice cells.

21. Transformed cells according to claim 19, further characterized as being cells of a dicotyledonous species, preferably soybean, alfalfa, rapeseed, sunflower, tobacco or tomato cells.

22. A maize cell or tissue culture comprising cells according to claim 21.

23. A transformed maize plant, the cells of which contain as foreign DNA at least one copy of the DNA sequence of an expression cassette according to claim 15.

24. A pharmaceutical composition in unit dosage form, comprising a therapeutically effective amount of Bandeiraea II lectin, chymopapain, or mixture thereof, in a non-toxic vehicle.

25. A composition according to claim 25 wherein the vehicle is adapted for systemic administration to a human or lower animal.

26. Bandeiraea II lectin, chymopapain, or a mixture thereof for use in a method of treatment or therapy of the human or animal body.

27. Use of Bandeiraea II lectin, chymopapain, or a mixture thereof for the manufacture of a medicament for the treatment of the human or animal body.

28. A composition for treatment of plant diseases caused by susceptible organisms, comprising a polypeptide selected from Bandeiraea II lectin and chymopapain or a mixture thereof, in a non-phytotoxic vehicle.

# EP 0 502 719 A1

**European Patent Office**

## PARTIAL EUROPEAN SEARCH REPORT

which under Rule 45 of the European Patent Convention shall be considered, for the purposes of subsequent proceedings, as the European search report

Application number

| | DOCUMENTS CONSIDERED TO BE RELEVANT | | EP 92301869.1 |
|---|---|---|---|

| Category | Citation of document with indication, where appropriate, of relevant passages | Relevant to claim | CLASSIFICATION OF THE APPLICATION (Int Cl⁵) |
|---|---|---|---|
| P,X | EP - A - 0 427 529 (PIONEER HI-BREED INTERNATIONAL INC.) * Abstract; claims * -- | 1, 6-12, 15-22 | A 01 N 63/00 A 01 N 65/00 C 12 N 15/29 C 12 N 15/05 C 12 N 5/04 A 61 K 37/02 |
| P,X | WO - A - 91/02 459 (UNIVERSITY OF FLORIDA) * Abstract; claims * -- | 1 | |
| A | PATENT ABSTRACTS OF JAPAN, unexamined applications, section C, vol. 13, no. 286, June 29, 1989 THE PATENT OFFICE JAPANESE GOVERNMENT page 129 C 613 * Kokai-no. 01-83 005 (SDS BIOTECH K.K.) * ---- | 1 | |

| TECHNICAL FIELDS SEARCHED (Int Cl⁵) |
|---|
| A 01 N C 12 N A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-22,24-28

Claims searched incompletely: —

Claims not searched: 23 (plant variety; Article 53 b) EPC)

Reason for the limitation of the search:

| Place of search | Date of completion of the search | Examiner |
|---|---|---|
| VIENNA | 18-05-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

11